(19) Europäisches Patentamt
European Patent Office
Office européen des brevets

(11) Veröffentlichungsnummer : **0 156 976**
**B1**

(12) **EUROPÄISCHE PATENTSCHRIFT**

(45) Veröffentlichungstag der Patentschrift : **30.09.87**

(21) Anmeldenummer : **84115022.0**

(22) Anmeldetag : **25.08.82**

(60) Veröffentlichungsnummer der früheren Anmeldung nach Art. 76 EPÜ : **0073061**

(51) Int. Cl.⁴ : **C 07 C119/06**, C 07 D317/28, C 07 D205/08, C 07 D405/14, C 07 D493/14, C 07 D403/04, C 07 D317/72, C 07 D317/36, C 07 D319/12

# (54) Optisch einheitliche Imine.

(30) Priorität : **27.08.81 CH 5524/81**
**25.06.82 CH 3925/82**

(43) Veröffentlichungstag der Anmeldung : **09.10.85 Patentblatt 85/41**

(45) Bekanntmachung des Hinweises auf die Patenterteilung : **30.09.87 Patentblatt 87/40**

(84) Benannte Vertragsstaaten :
**AT BE CH DE FR GB IT LI LU NL SE**

(56) Entgegenhaltungen :
**GB-A- 2 014 145**

(73) Patentinhaber : **F. HOFFMANN-LA ROCHE & CO. Aktiengesellschaft**
**CH-4002 Basel (CH)**

(72) Erfinder : **Hubschwerlen, Christian Nicolas, Dr.**
**Rue de la Gendarmerie**
**F-68200 Durmenach (FR)**
Erfinder : **Schmid, Gérard, Dr.**
**Mittlerer Feldweg 6**
**CH-4468 Kienberg (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue optisch einheitliche Imine der allgemeinen Formel

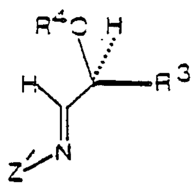

(III)

worin $R^3$ niederes Alkyl, Phenyl-niederes Alkyl, niederes Alkoxy-niederes Alkyl, insbesondere niederes Alkoxymethyl, und $R^4$ niederes Alkyl oder Phenyl-niederes Alkyl oder $R^3$ und $R^4$ zusammen mit dem Chiralitätszentrum einen 5- oder 6-gliedrigen, gegebenenfalls ein weiteres, mit dem Chiralitätszentrum nicht direkt verbundenes Sauerstoffatom enthaltenden O-Heterocyclus, der gegebenenfalls durch niederes Alkyl, niederes Alkoxy, Oxo oder Spirocyclo-niederes Alkyl substituiert sein kann, wobei benachbarte niedere Alkoxygruppen zusammen einen Ring bilden können, wie z. B. bei Isopropylidendioxy, und Z' 2,4- oder 3,4-Di(niederes Alkoxy)-benzyl, 2,4- oder 3,4-Di(niederes Alkoxy) phenyl, Di [4-(niederes Alkoxy) phenyl] methyl oder 4-(niederes Alkoxy) phenyl bedeuten,
und die entsprechenden optischen Antipoden davon.

Gegenstand der vorliegenden Erfindung sind optisch einheitliche Verbindungen der obigen Formel III und die entsprechenden Antipoden davon und die Herstellung dieser Verbindungen. Die erfindungsgemässen Stoffe können zur Herstellung von optisch einheitlichen β-Lactamen der weiter unten definierten Formel Ia verwendet werden.

Der Ausdruck « niederes Alkyl » für sich allein genommen, oder in Kombinationen, wie « niederes Alkoxy » und dergleichen, bezeichnet geradkettige oder verzweigte gesättigte Kohlenwasserstoffreste mit höchstens 7, vorzugsweise höchstens 4 Kohlenstoffatomen, wie Methyl, Aethyl, Isopropyl und dergleichen.

Beispiele für 2,4- und 3,4-Di(niederes Alkoxy) benzyl sind insbesondere 2,4- und 3,4-Dimethoxybenzyl. Beispiele für 2,4- und 3,4-Di(niederes Alkoxy) phenyl sind insbesondere 2,4- und 3,4-Dimethoxyphenyl. Ein Beispiel für Di [4-(niederes Alkoxy) phenyl] methyl ist insbesondere Di(4-methoxyphenyl) methyl. Ein Beispiel für 4-(niederes Alkoxyl) phenyl ist insbesondere 4-Methoxyphenyl.

In einer besonders bevorzugten Ausführungsform bedeuten $R^3$ und $R^4$ zusammen mit dem Chiralitätszentrum eine Gruppe der Formel :

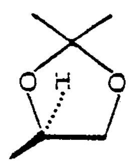 , 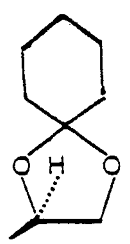 , 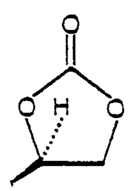 , 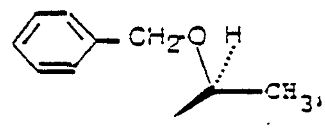

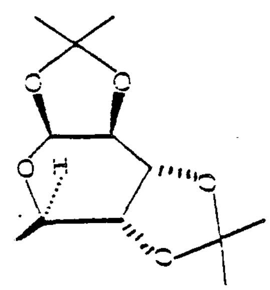 , 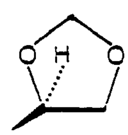 oder 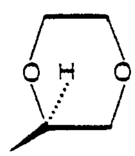

In einer speziellen Ausführungsform umfasst die vorliegende Erfindung Verbindungen der allgemeinen Formel

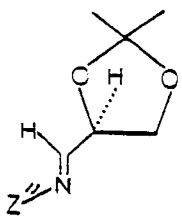

(IIIa)

worin Z″ 2,4- oder 3,4-Di(niederes Alkoxy) benzyl, Di [4-(niederes Alkoxy) phenyl] methyl oder 4-(niederes Alkoxy) phenyl bedeutet,
und die entsprechenden optischen Antipoden davon.

Z″ bedeutet vorzugsweise 2,4-Dimethoxybenzyl.

Die nachfolgend aufgeführten Verbindungen sind Vertreter der durch die obige Formel III definierten Verbindungsklasse :

Isopropyliden-L-glyceraldehyd-(2,4-dimethoxybenzyl) imin und
Isopropyliden-L-glyceraldehyd-(3,4-dimethoxybenzyl) imin.

Die Verbindungen der Formel III und deren optische Antipoden können erfindungsgemäss hergestellt werden, indem man einen Aldehyd der allgemeinen Formel

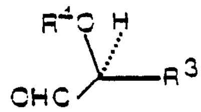

(IV)

worin $R^3$ und $R^4$ obige Bedeutung besitzen, oder den optischen Antipoden davon mit einem Amin der allgemeinen Formel

$$H_2N—Z' \qquad (V)$$

worin Z′ obige Bedeutung besitzt, umsetzt. Man arbeitet dabei vorzugsweise in einem inerten organischen Lösungsmittel, beispielsweise in einem halogenierten Kohlenwasserstoff, wie Methylenchlorid, Chloroform, 1,2-Dichloräthan und dergleichen, oder in einem Kohlenwasserstoff, wie Benzol, Toluol und dergleichen. Vorzugsweise entfernt man dabei laufend das während der Reaktion entstehende Reaktionswasser, beispielsweise durch azeotrope Destillation oder durch Arbeiten in Gegenwart eines wasserentziehenden Mittels, beispielsweise in Gegenwart eines geeigneten Molekularsiebes, oder von anderen herkömmlichen Trocknungsmitteln, wie Kaliumcarbonat, Magnesiumsulfat und dergleichen. Bei azeotroper Entfernung des gebildeten Reaktionswassers arbeitet man bei der Siedetemperatur des gewählten Lösungsmittels ; bei Verwendung eines wasserentziehenden Mittels arbeitet man vorzugsweise bei Raumtemperatur.

Die Verbindungen der Formel III und ihre optischen Antipoden können in Gegenwart einer Base mit einem reaktiven Derivat einer Carbonsäure der allgemeinen Formel

$$R^{11}—CH_2—COOH \qquad (II)$$

worin $R^{11}$ Azido, Phthalimido oder die Gruppe $ROCO—CH=C(CH_3)—NH—$ und R niederes Alkyl bedeuten, umgesetzt werden, wobei man ein optisch einheitliches β-Lactam der allgemeinen Formel

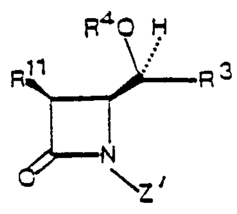

(Ia)

worin $R^{11}$, $R^3$, $R^4$ und Z′ obige Bedeutung besitzen, oder den optischen Antipoden davon erhält.

Bei der Reaktion eines reaktiven Derivates einer Carbonsäure der Formel II mit einer Verbindung der Formel III bzw. dem optischen Antipoden davon handelt es sich um eine dem Fachmann geläufige Cycloaddition. Geeignete reaktive Carbonsäurederivate sind beispielsweise die entsprechenden Carbonsäurehalogenide, insbesondere die Carbonsäurechloride, entsprechende Carbonsäureanhydride und gemischte Anhydride (z. B. mit Trifluoressigsäure, Mesitylensulfonsäure und dergleichen), entsprechende Carbonsäureimidazolide und dergleichen. Zweckmässigerweise arbeitet man dabei in Gegenwart eines tertiären Amins, wie Triäthylamin, und in einem inerten organischen Lösungsmittel, wobei insbesondere Aether, wie Tetrahydrofuran, Diäthyläther, t-Butylmethyläther, Dioxan, Aethylenglykoldimethyläther oder dergleichen, halogenierte Kohlenwasserstoffe, wie Methylenchlorid, Chloroform, 1,2-Dichloräthan oder dergleichen, Acetonitril, Dimethylformamid oder dergleichen in Frage kommen. Die obige Cycloaddition wird dabei in einem Temperaturbereich von etwa — 30 °C bis etwa 50 °C durchgeführt.

Die obige Reaktion einer Verbindung der Formel II mit einer optisch einheitlichen Verbindung der Formel III oder dem optischen Antipoden davon liefert eine Verbindung der obigen Formel Ia bzw. den entsprechenden optischen Antipoden davon, wobei die Substituenten in 3- und 4-Stellung des Azetidinringes zueinander wie erwartet cis-ständig sind. Ueberraschenderweise hat es sich jedoch gezeigt, dass die Verwendung einer optisch aktiven Verbindung der Formel III bzw. des optischen Antipoden davon in der obigen Cycloaddition in hoher optischer Ausbeute zwei neue optische Zentren induziert, d. h. mit hoher Diastereoselektivität zur Bildung von nur einem von zwei möglichen diastereomeren Produkten führt. Im erhaltenen Produkt konnte das zweite mögliche cis-Cycloadditionsprodukt, das zum tatsächlich erhaltenen Produkt diastereoisomer wäre, nicht nachgewiesen werden.

Die Verbindungen der Formel III und ihre entsprechenden optischen Antipoden, welche in der oben beschriebenen Cycloaddition überraschenderweise nur das eine von zwei diastereomeren Produkten liefern, müssen niche isoliert werden, sondern können direkt der erfindungsgemässen Cycloaddition unterworfen werden.

Die optisch einheitlichen β-Lactame der Formel Ia und deren Herstellung durch Umsetzen einer Verbindung der Formel III mit einem reaktiven Derivat einer Carbonsäure der Formel II werden in der Europäischen Patentanmeldung Nr. 82.107790.6 (Veröffentlichungsnummer : 73.061) beansprucht.

Beispiel 1

Zu einer bei Raumtemperatur gerührten Lösung von 4,9 g (37,65 mMol) Isopropyliden-D-glyceraldehyd in 100 ml trockenem Methylenchlorid (frei von Methanol) gibt man 10 g Molekularsieb 4 Å und anschliessend tropfenweise eine Lösung von 6,29 g (37,65 mMol) 2,4-Dimethoxybenzylamin in 20 ml trockenem Methylenchlorid. Man rührt die Reaktionsmischung während 2 Stunden bei Raumtemperatur, versetzt anschliessend mit 5 g wasserfreiem Magnesiumsulfat, rührt noch während 30 Minuten und filtriert anschliessend, wobei der Filterkuchen noch mit 20 ml Methylenchlorid gewaschen wird.

Die erhaltene organische Lösung von Isopropyliden-D-glyceraldehyd-(2,4-dimethoxybenzyl) imin wird unter Argon auf — 18° gekühlt und unter Rühren mit 5,5 ml (39,5 mMol) Triäthylamin versetzt. Nach wenigen Minuten gibt man über einen Zeitraum von 1 Stunde eine Lösung von 8,8 g (39,3 mMol) Phthaloylglycylchlorid in 100 ml trockenem Methylenchlorid dazu, rührt die Reaktionsmischung während 2 Stunden bei — 15° und lässt anschliessend auf Raumtemperatur erwärmen. Man rührt noch über Nacht bei Raumtemperatur, wäscht die Reaktionsmischung dreimal mit je 100 ml Wasser und mit 100 ml Kochsalzlösung und trocknet die erhaltene Lösung über Natriumsulfat. Man dampft ein und chromatographiert den erhaltenen Rückstand an einer Kieselgelsäule (Korngrösse : 230-400 mesh) unter Eluieren mit Hexan/Essigester (1 : 1). Man erhält 15,8 g (90 %) N-[(3R,4R)-cis-1-(2,4-Dimethoxybenzyl)-4-[(S)-2,2-dimethyl-1,3-dioxolan-4-yl]-2-oxo-3-azetidinyl] phthalimid als Schaum ; $[\alpha]_D$ = — 37° (c = 1 in Chloroform) ; MS : 466 ($M^+$). Nach der Umkristallisation aus Aethylacetat erhält man farblose Kristalle vom Schmelzpunkt 155° ; $[\alpha]_D$ = — 49,2 (c = 0,8 in Chloroform).

Beispiel 2

Zu einer bei Raumtemperatur gerührten Lösung von 0,9 g (5,4 mMol) 2,4-Dimethoxybenzylamin in 100 ml Methyllenchlorid gibt man 3 g Molekularsieb 4 Å und nach 20 Minuten 0,7 g (5,4 mMol) Isopropyliden-L-glyceraldehyd und 5 g wasserfreies Magnesiumsulfat. Anschliessend rührt man noch während 1 Stunde bei Raumtemperatur. Die erhaltene organische Lösung von Isopropyliden-L-glyceraldehyd-(2,4-dimethoxybenzyl) imin wird unter Argon auf — 20° gekühlt und unter Rühren mit 0,88 ml (5,4 mMol) Triäthylamin versetzt. Dann wird innerhalb 1 Stunde eine Lösung von 1,25 g (5,6 mMol) Phthaloylglycylchlorid in 20 ml trockenem Methylenchlorid zugetropft und anschliessend über Nacht bei Raumtemperatur weiter gerührt. Man arbeitet auf wie in Beispiel 1 beschrieben und erhält nach der Chromatographie 1,77 g (70 %) N-[(3S,4S)-cis-1-(2,4-Dimethoxybenzyl)-4-[(R)-2,2-dimethyl-1,3-dioxolan-4-yl]-2-oxo-3-azetidinyl]-phthalimid als Schaum ; $[\alpha]_D$ = + 41° (c = 0,8 in Chloroform) ; MS : 466 ($M^+$). Nach dem Umkristallisieren aus Aethylacetat erhält man ein kristallines Produkt mit $[\alpha]_D$ = + 48° (c = 0,6 in Chloroform).

Beispiel 3

1 g (1,2 : 3,4)-di-O-Isopropyliden-α-D-galacto-hexodialdo-1,5-pyranose (3,87 mMol) werden in 60 ml absolutem Methylenchlorid, enthaltend 5 g Molekularsieb 4 Å, unter Rühren in einer Argonatmosphäre gelöst. Die Lösung wird bei Zimmertemperatur mit einer Lösung von 0,68 g 2,4-Dimethoxybenzylamin (4 mMol) in 10 ml Methylenchlorid tropfenweise behandelt. Die Lösung wird bei Zimmertemperatur 30 Minuten gerührt, anschliessend auf — 20° abgekühlt und mit 0,65 ml (0,47 g, 4,6 mMol) Triäthylamin behandelt und nach einigen Minuten mit einer Lösung von 0,8 g (0,40 mMol) Phthaloylglycylchlorid in 20 ml Methylenchlorid. Das Reaktionsgemisch wird auf Zimmertemperatur erwärmt und anschliessend 1,5 Stunden gerührt. Nach Filtration wird die erhaltene organische Lösung mit Wasser (2 mal 150 ml) und wässriger Kochsalzlösung (1 mal 100 ml) gewaschen und über Magnesiumsulfat getrocknet. Nach Eindampfen des Lösungsmittels und chromatographischer Reinigung an Kieselgel (230-400 mesh ; n-Hexan : Aethylacetat 1 : 1 als Lösungsmittel) erhält man 1,8 g (75 %) N-[cis-1-(2,4-Dimethoxybenzyl)-2-oxo-4-[(3aαH,8bαH)-tetrahydro-2,2,7,7-tetramethyl-5H-bis [1,3] dioxolo [4,5-b : 4', 5'-d] pyran-5-yl]-3-azetidinyl]-phthalimid als Schaum. $[\alpha]_D$ = — 27° (c = 1 % in Methanol) MS : 594 ($M^+$). IR 1767, 1722 $cm^{-1}$ (KBr).

Beispiel 4

2,0 g (11,76 mMol) Cyclohexyliden-D-glyceraldehyd werden in 8 ml absolutem Methylenchlorid gelöst und bei Zimmertemperatur mit 2,06 g (12,35 mMol) 2,4-Dimethoxybenzylamin behandelt. Nach 30-minütigem Rühren wird das Reaktionsgemisch auf 0° abgekühlt und mit 1,96 ml (14,11 mMol) Triäthylamin und tropfenweise mit einer Lösung von 2,76 g (12,35 mMol) Phthaloylglycylchlorid in 4 ml Methylenchlorid behandelt. Das Reaktionsgemisch wird 12 Stunden bei Zimmertemperatur gerührt, anschliessend mit 100 ml Methylenchlorid verdünnt und nacheinander mit 30 ml Wasser, 30 ml 0,1N wässriger Salzsäure, 30 ml Wasser und 30 ml wässriger Kochsalzlösung gewaschen und über Natriumsulfat getrocknet. Das Lösungsmittel wird abgedampft und der Rückstand an Kieselgel chromatographisch gereinigt (230-400 mesh ; Aethylacetat : n-Hexan 1 : 1 als Lösungsmittel). Man erhält 4,3 g (72 %) N-[(3R,4R)-1-(2,4-Dimethoxybenzyl)-4-[(S)-1,4-dioxaspiro [4.5] dec-2-yl]-2-oxo-3-azetidinyl]-phthalimid, $[\alpha]_D$ = — 18° (c = 0,5 in Methanol).
Elementaranalyse : berechnet für

| | | | |
|---|---|---|---|
| $C_{28}H_{30}N_2O_7$ : | C 66,39, | H 5,97, | N 5,53 % |
| gef. | C 65,94, | H 5,94, | N 5,57 % |

IR (KBr) $cm^{-1}$ : 3435, 1768, 1721, 1589, 1508, 1208.

NMR ($CDCl_3$)δ (ppm) : 1,2-1,6 (10H, m, $(CH_2)_5$), 3,35 (1H, dd, 6 und 9 Hz, H-4), 3,5-3,8 (2H, m, O—$CH_2$) 3,81 und 3,82 (6H, 2s, 2 × $OCH_3$), 4,20 (1H, m, O—CH), 4,15 und 4,90 (2H, 2d, 14 Hz, N—$CH_2$-Ar), 5,25 (1H, d, 6 Hz, $H_3$), 6,50 (2H, m, 2H Ar), 7,27 (1H, d, Ar), 7,85 (4H, m, Ar).
MS : $M^+$ : 506.

Beispiel 5

Zu einer bei Raumtemperatur gerührten Lösung von 0,9 g (5,4 mMol) 3,4-Dimethoxybenzylamin in 100 ml Methylenchlorid gibt man 3 g Molekularsieb 4 Å und nach 20 Minuten 0,7 g (5,4 mMol) Isopropyliden-L-glyceraldehyd und 5 g wasserfreies Magnesiumsulfat. Anschliessend rührt man noch während 1 Stunde bei Raumtemperatur. Die erhaltene organische Lösung von Isopropyliden-L-glyceraldehyd-(3,4-dimethoxybenzyl) imin wird unter Argon auf — 20° gekühlt und unter Rühren mit 0,88 ml (5,4 mMol) Triäthylamin versetzt. Dann wird innerhalb 1 Stunde eine Lösung von 1,25 g (5,6 mMol) Phthaloylglycylchlorid in 20 ml trockenem Methylenchlorid zugetropft und anschliessend über Nacht bei Raumtemperatur weiter gerührt. Man arbeitet auf wie in Beispiel 1 beschrieben und erhält nach der Chromatographie 1,56 g (62 %) N-(3S,4S)-cis-1-(3,4-Dimethoxybenzyl)-4-[(R)-2,2-dimethyl-1,3-dioxolan-4-yl]-2-oxo-3-azetidinyl] phthalimid als Schaum ; $[\alpha]_D$ = + 16,2° (c = 1,0 in Methanol) ;
MS : 466 ($M^+$).
Elementaranalyse : berechnet für

| | | | |
|---|---|---|---|
| $C_{25}H_{26}N_2O_7$ : | C 64,37, | H 5,62, | N 6,01 % |
| gef. | C 63,53, | H 5,72, | N 5,96 % |

IR (KBr $cm^{-1}$) : 3430, 1766, 1721, 1609, 1517

Beispiel 6

Zu einer bei Raumtemperatur gerührten Lösung von 670 mg (4 mMol) 2,4-Dimethoxybenzylamin in 70 ml Methylenchlorid gibt man 2,5 g Molekularsieb 4 Å und nach 20 Minuten 660 mg (4 mMol) (S)-2-Benzyloxypropionaldehyd und 3,7 g wasserfreies Magnesiumsulfat. Anschliessend rührt man 1 Stunde bei Raumtemperatur. Die erhaltene Lösung wird in einer Argonatmosphäre auf — 20° gekühlt und unter Rühren mit 0,65 ml (4 mMol) Triäthylamin versetzt. Dann wird innerhalb 1 Stunde eine Lösung von 930 mg (4,15 mMol) Phthaloylglycylchlorid in 15 ml trockenem Methylenchlorid zugetropft und anschliessend 12 Stunden bei Raumtemperatur weiter gerührt. Man arbeitet auf wie in Beispiel 1 beschrieben und erhält nach der Chromatographie 990 mg (50 %) N-[(3S,4S)-4-[(S)-1-(Benzyloxy) äthyl]-1-(2,4-dimethylbenzyl)-2-oxo-3-azetidinyl] phthalimid als Schaum. $[\alpha]_D$ = + 44.6 (c = 1 in Chloroform).
MS : = 500 ($M^+$).
Elementaranalyse : berechnet für

| | | | |
|---|---|---|---|
| $C_{29}H_{28}N_2O_6$ : | C 69,59, | H 5,64, | N 5,60 % |
| gef. | C 69,06, | H 5,80, | N 5,36 % |

IR (KBr) $cm^{-1}$ : 1766, 1721, 1613, 1589, 1508.

**Patentansprüche**

1. Optisch einheitliche Verbindungen der allgemeinen Formel

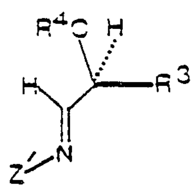

(III)

worin $R^3$ niederes Alkyl, Phenyl-niederes Alkyl, niederes Alkoxy-niederes Alkyl, insbesondere niederes Alkoxymethyl, und $R^4$ niederes Alkyl oder Phenyl-niederes Alkyl oder $R^3$ und $R^4$ zusammen mit dem Chiralitätszentrum einen 5- oder 6-gliedrigen, gegebenenfalls ein weiteres, mit dem Chiralitätszentrum nicht direkt verbundenes Sauerstoffatom enthaltenden O-Heterocyclus, der gegebenenfalls durch niederes Alkyl, niederes Alkoxy, Oxo oder Spirocyclo-niederes Alkyl substituiert sein kann, wobei benachbarte niedere Alkoxygruppen zusammen einen Ring bilden können, wie z. B. bei Isopropylidendioxy, und Z' 2,4- oder 3,4-Di(niederes Alkoxy)-benzyl, 2,4- oder 3,4-Di(niederes Alkoxy) phenyl, Di [4-(niederes Alkoxy) phenyl] methyl oder 4-(niederes Alkoxy) phenyl bedeuten, und die mit nieder bezeichneten Reste bis zu 7 Kohlenstoffatome besitzen,
und die entsprechenden optischen Antipoden davon.

2. Verbindungen gemäss Anspruch 1, dadurch gekennzeichnet, dass $R^3$ und $R^4$ zusammen mit dem Chiralitätszentrum eine Gruppe der Formel

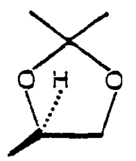 , 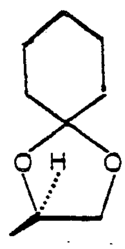 ; 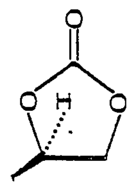 , 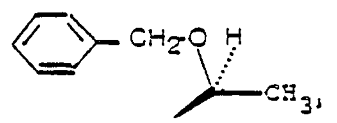

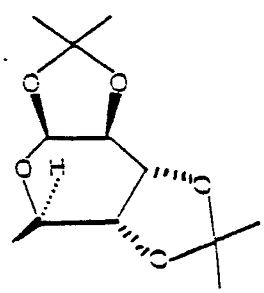 , 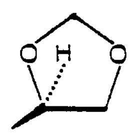 oder 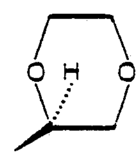

bedeuten.

3. Verbindungen gemäss Anspruch 1 der allgemeinen Formel

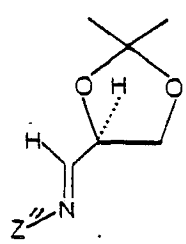

(IIIa)

worin Z" 2,4- oder 3,4-Di(niederes Alkoxy) benzyl, Di [4-(niederes Alkoxy) phenyl] methyl oder 4-(niederes Alkoxy) phenyl bedeutet,
und die entsprechenden optischen Antipoden davon.

4. Isopropyliden-L-glyceraldehyd-(2,4-dimethoxybenzyl)-imin.

5. Isopropyliden-L-glyceraldehyd-(3,4-dimethoxybenzyl)-imin.

6. Verfahren zur Herstellung von Verbindungen der allgemeinen Formel

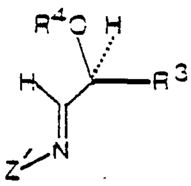

(III)

worin $R^3$ niederes Alkyl, Phenyl-niederes Alkyl, niederes Alkoxy-niederes Alkyl, insbesondere niederes Alkoxymethyl, und $R^4$ niederes Alkyl oder Phenyl-niederes Alkyl oder $R^3$ und $R^4$ zusammen mit dem Chiralitätszentrum einen 5- oder 6-gliedrigen, gegebenenfalls ein weiteres, mit dem Chiralitätszentrum nicht direkt verbundenes Sauerstoffatom enthaltenden O-Heterocyclus, der gegebenenfalls durch niederes Alkyl, niederes Alkoxy, Oxo oder Spirocyclo-niederes Alkyl substituiert sein kann, wobei benachbarte niedere Alkoxygruppen zusammen einen Ring bilden können, wie z. B. bei Isopropylidendioxy, und Z' 2,4- oder 3,4-Di(niederes Alkoxy) benzyl, 2,4- oder 3,4-Di(niederes Alkoxy) phenyl, Di [4-(niederes Alkoxy) phenyl] methyl oder 4-(niederes Alkoxy) phenyl bedeuten, und die mit nieder bezeichneten Reste bis zu 7 Kohlenstoffatome besitzen,
und der entsprechenden optischen Antipoden, dadurch gekennzeichnet, dass man einen Aldehyd der allgemeinen Formel

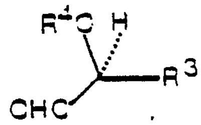

(IV)

worin $R^3$ und $R^4$ obige Bedeutung besitzen,
oder den optischen Antipoden davon mit einem Amin der allgemeinen Formel

$$H_2N—Z' \qquad (V)$$

worin Z' obige Bedeutung besitzt, umsetzt.

7. Verfahren nach Anspruch 6, dadurch gekennzeichnet, dass man als Ausgangsstoff eine Verbindung der Formel IV, worin $R^3$ und $R^4$ zusammen mit dem Chiralitätszentrum eine Gruppe der Formel

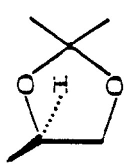 , 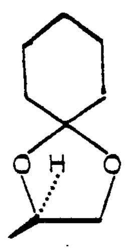 ; 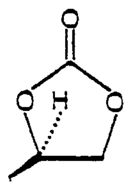 , 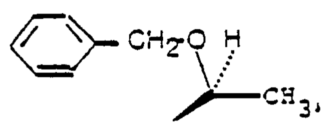

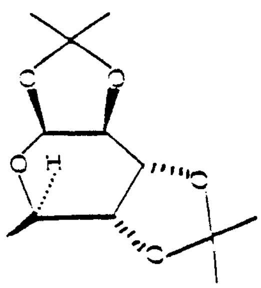 , 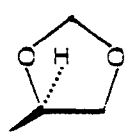 oder 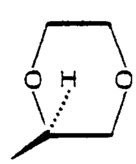

bedeuten, oder den optischen Antipoden davon verwendet.

8. Verfahren nach Anspruch 6, dadurch gekennzeichnet, dass man Isopropyliden-L-glyceraldehyd oder den optischen Antipoden davon mit einem 2,4- oder 3,4-Di(niederen Alkoxy)-benzyl-, einem Di [4-(niederen Alkoxy) phenyl] methyl- oder einem 4-(niederen Alkoxy) phenylamin umsetzt.

9. Verfahren nach Anspruch 6, dadurch gekennzeichnet, dass man Isopropyliden-L-glyceraldehyd mit 2,4-Dimethoxybenzylamin umsetzt.

10. Verfahren nach Anspruch 6, dadurch gekennzeichnet, dass man Isopropyliden-L-glyceraldehyd mit 3,4-Dimethoxybenzylamin umsetzt.

## Claims

1. Optically uniform compounds of the general formula

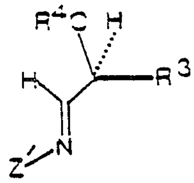

(III)

wherein $R^3$ signifies lower alkyl, phenyl-lower alkyl, lower alkoxy-lower alkyl, especially lower alkoxymethyl, and $R^4$ signifies lower alkyl or phenyl-lower alkyl or $R^3$ and $R^4$ together with the centre of chirality signify a 5- or 6-membered O-heterocycle which optionally contains a further oxygen atom not directly linked with the centre of chirality and which can be substituted, if desired, by lower alkyl, lower alkoxy, oxo or spirocyclo-lower alkyl, whereby adjacent lower alkoxy groups can together form a ring, such as e. g. in the case of isopropylidenedioxy, and Z′ signifies 2,4- or 3,4-di(lower alkoxy) benzyl, 2,4- or 3,4-di(lower alkoxy) phenyl, di [4-(lower alkoxy) phenyl] methyl or 4-(lower alkoxy) phenyl, and the residues denoted by lower have up to 7 carbon atoms,
and the corresponding optical antipodes thereof.

2. Compounds in accordance with claim 1, characterized in that $R^3$ and $R^4$ together with the centre of chirality signify a group of the formula

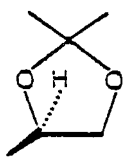 , 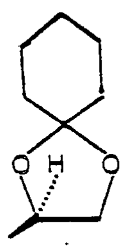 ; 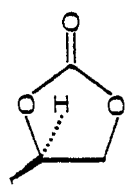 , 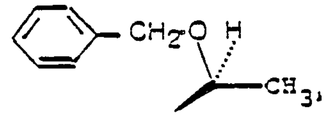 ,

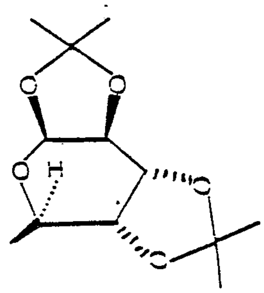 , 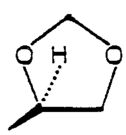 or 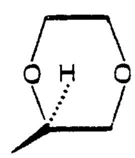

3. Compounds in accordance with claim 1 of the general formula

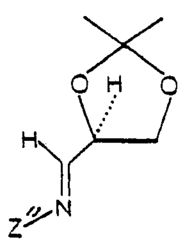

(IIIa)

wherein Z″ signifies 2,4- or 3,4-di(lower alkoxy) benzyl, di [4-(lower alkoxy) phenyl] methyl or 4-(lower alkoxy) phenyl,
and the corresponding optical antipodes thereof.

4. Isopropylidene-L-glyceraldehyde (2,4-dimethoxybenzyl) imine.

5. Isopropylidene-L-glyceraldehyde (3,4-dimethoxybenzyl) imine.

6. A process for the manufacture of compounds of the general formula

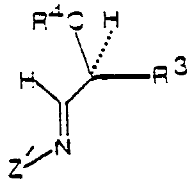

(III)

wherein $R^3$ signifies lower alkyl, phenyl-lower alkyl, lower alkoxy-lower alkyl, especially lower alkoxymethyl, and $R^4$ signifies lower alkyl or phenyl-lower alkyl or $R^3$ and $R^4$ together with the centre of chirality signify a 5- or 6-membered O-heterocycle which optionally contains a further oxygen atom not directly linked with the centre of chirality and which can be substituted, if desired, by lower alkyl, lower alkoxy, oxo or spirocyclo-lower alkyl, whereby adjacent lower alkoxy groups can together form a ring, such as e. g. in the case of isopropylidenedioxy, and Z' signifies 2,4- or 3,4-di(lower alkoxy) benzyl, 2,4- or 3,4-di(lower alkoxy) phenyl, di[4-(lower alkoxy) phenyl] methyl or 4-(lower alkoxy) phenyl, and the residues denoted by lower have up to 7 carbon atoms,
and of the corresponding optical antipodes, characterized by reacting an aldehyde of the general formula

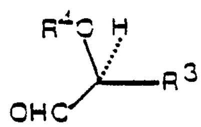

(IV)

wherein $R^3$ and $R^4$ have the above significance,
or the optical antipode thereof with an amine of the general formula

$$H_2N—Z' \qquad (V)$$

wherein Z' has the above significance.

7. A process according to claim 6, characterized in that a compound of formula IV in which $R^3$ and $R^4$ together with the centre of chirality signify a group of the formula

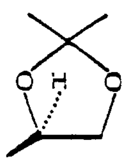 , 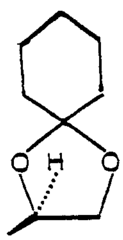 ; 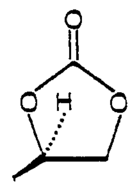 , 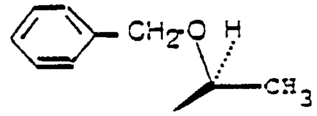

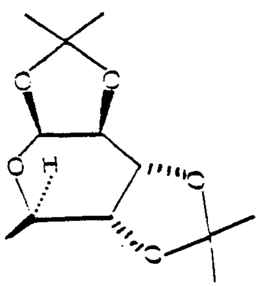 , 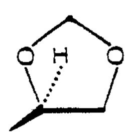 or 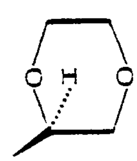

or the optical antipode thereof is used as the starting material.

8. A process according to claim 6, characterized in that isopropylidene-L-glyceraldehyde or the optical antipode thereof is reacted with a 2,4- or 3,4-di(lower alkoxy)-benzylamine, a di [4-(lower alkoxy) phenyl] methylamine or a 4-(lower alkoxy) phenylamine.

9. A process according to claim 6, characterized in that isopropylidene-L-glyceraldehyde is reacted with 2,4-dimethoxybenzylamine.

10. A process according to claim 6, characterized in that isopropylidene-L-glyceraldehyde is reacted with 3,4-dimethoxybenzylamine.

**Revendications**

1. Composés, à l'état d'isomères optiquement uniformes, répondant à la formule générale

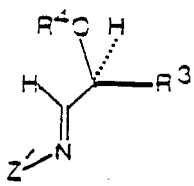

(III)

dans laquelle $R^3$ représente un groupe alkyle inférieur, phényl-alkyle inférieur, (alcoxy inférieur)-alkyle inférieur, en particulier (alcoxy inférieur)-méthyle, et $R^4$ représente un groupe alkyle inférieur ou phényl-alkyle inférieur, ou bien $R^3$ et $R^4$ forment ensemble et avec le centre de chiralité, un hétérocycle oxygéné à 5 ou 6 chaînons contenant éventuellement un autre atome d'oxygène non relié directement au centre de chiralité, et qui peut, le cas échéant, être substitué par un groupe alkyle inférieur, alcoxy inférieur, oxo ou spirocycloalkyle inférieur, les groupes alcoxy inférieurs voisins pouvant former ensemble un cycle, par exemple sous la forme d'un groupe isopropylidène-dioxy, et Z' représente un groupe 2,4- or 3,4-di-(alcoxy inférieur)-benzyle, 2,4- ou 3,4-di-(alcoxy inférieur)-phényle, di-[4-(alcoxy inférieur) phényl]-méthyl ou 4-(alcoxy inférieur)-phényle, les groupes qualifiés d'« inférieurs » contenant jusqu'à 7 atomes de carbone, et leurs antipodes optiques.

2. Composés selon la revendication 1, caractérisés en ce que $R^3$ et $R^4$ forment ensemble et avec le centre de chiralité, un groupe de formule

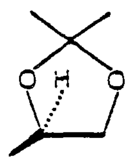 , 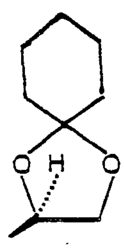 ; 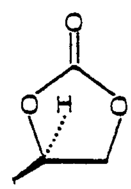 , 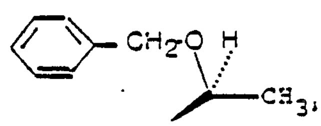

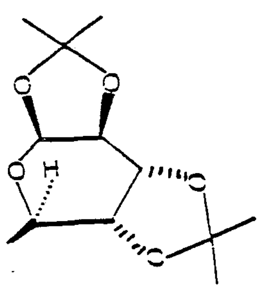 , 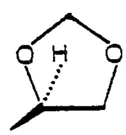 ou 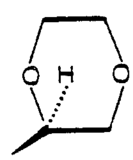

3. Composés selon la revendication 1, répondant à la formule générale

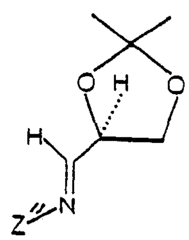

(IIIa)

dans laquelle Z'' représente un groupe 2,4- ou 3,4-di-(alcoxy inférieur)-benzyle, di-[4-(alcoxy inférieur)-phényl]-méthyle ou 4-(alcoxy inférieur)-phényle,
et les antipodes optiques correspondants.

4. L'isopropylidène-L-glycéraldéhyde-(2,4-diméthoxybenzyl)-imine.

5. L'isopropylidène-L-glycéraldéhyde-(3,4-diméthoxybenzyl)-imine.

6. Procédé de préparation des composés de formule générale

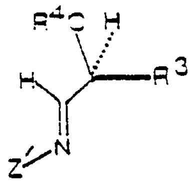

(III)

dans laquelle $R^3$ représente un groupe alkyle inférieur, phényl-alkyle inférieur, (alcoxy inférieur)-alkyle inférieur, en particulier (alcoxy inférieur)-méthyle et $R^4$ représente un groupe alkyle inférieur ou phényl-alkyle inférieur ou bien $R^3$ et $R^4$ forment ensemble et avec le centre de chiralité un hétérocycle oxygéné à 5 ou 6 chaînons contenant, le cas échéant, un autre atome d'oxygène non relié directement au centre de chiralité et qui peut, le cas échéant, être substitué par un groupe alkyle inférieur, alcoxy inférieur, oxo ou spirocyclo-alkyle inférieur, des groupes alcoxy inférieurs voisins pouvant former ensemble un cycle, sous la forme par exemple d'un groupe isopropylidène-dioxy, et Z' représente un groupe 2,4- ou 3,4-di-(alcoxy inférieur)-benzyle, 2,4- ou 3,4-di-(alcoxy inférieur)-phényle, di-[4-(alcoxy inférieur)-phényl]-méthyle ou 4-(alcoxy inférieur)-phényle, les groupes qualifiés d'« inférieurs » contenant jusqu'à 7 atomes de carbone, et des antipodes optiques correspondants, caractérisé en ce que l'on fait réagir un aldéhyde de formule générale

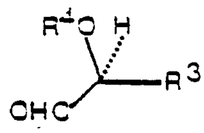

(IV)

dans laquelle $R^3$ et $R^4$ ont les significations indiquées ci-dessus, ou son antipode optique, avec une amine de formule générale

$$H_2N—Z' \quad (V)$$

dans laquelle Z' a les significations indiquées ci-dessus.

7. Procédé selon la revendication 6, caractérisé en ce que l'on utilise en tant que produit de départ un composé de formule IV dans laquelle $R^3$ et $R^4$ forment ensemble et avec le centre de chiralité, un groupe de formule

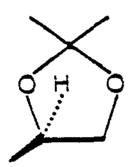 , 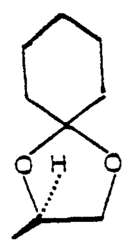 ; 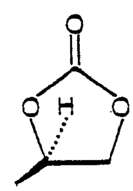 , 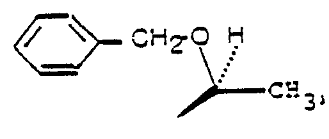

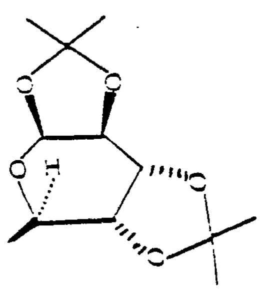 , 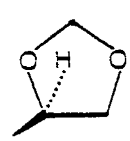 ou 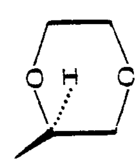

ou l'antipode optique d'un tel composé.

8. Procédé selon la revendication 6, caractérisé en ce que l'on fait réagir l'isopropylidène-L-glycéraldéhyde ou l'antipode optique de ce composé avec une 2,4- ou 3,4-di-(alcoxy inférieur)-benzyl-, une di-[4-(alcoxy inférieur)-phényl]-méthyl- ou une 4-(alcoxy inférieur)-phénylamine.

9. Procédé selon la revendication 6, caractérisé en ce que l'on fait réagir l'isopropylidène-L-glycéraldéhyde avec la 2,4-diméthoxybenzylamine.

10. Procédé selon la revendication 6, caractérisé en ce que l'on fait réagir l'isopropylidène-L-glycéraldéhyde avec la 3,4-diméthoxybenzylamine.